(19) Europäisches Patentamt
European Patent Office
Office européen des brevets

(11) **EP 4 209 232 A1**

(12) **EUROPEAN PATENT APPLICATION**

(43) Date of publication:
**12.07.2023 Bulletin 2023/28**

(21) Application number: **22150470.7**

(22) Date of filing: **06.01.2022**

(51) International Patent Classification (IPC):
**A61L 27/24** *(2006.01)* **A61L 27/36** *(2006.01)*
**A61L 27/56** *(2006.01)*

(52) Cooperative Patent Classification (CPC):
**A61L 27/24; A61L 27/3604; A61L 27/3654;
A61L 27/3687; A61L 27/56;** A61L 2430/06

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Geistlich Pharma AG
6110 Wolhusen (CH)**

(72) Inventors:
• **STIEFEL, Niklaus
6003 Luzern (CH)**
• **ZIHLMANN, Carla
6170 Schüpfheim (CH)**

(74) Representative: **Savatier, Yves
Geistlich Pharma AG
Bahnhofstrasse 40
6110 Wolhusen (CH)**

(54) **COLLAGEN DEVICE FOR MENISCUS REGENERATION**

(57)    The invention relates to:
- A collagen device for meniscus regeneration comprising a resilient resorbable chemically crosslinked collagen sponge wherein the external surface of the resilient resorbable chemically crosslinked collagen sponge is partially or totally covered by a dense layer of collagen fibers that is impermeable to meniscal cells, said collagen device for meniscus regeneration having an onset temperature of 40 to 70 °C and showing by mercury intrusion porosimetry interconnected pores with at least 60 % porosity with a pore diameter more than 10 μm,
- A process for preparing that collagen device for meniscus regeneration, and
- the use of that collagen device in the knee region as an implant into a tear or segmental defect of the meniscus to be sutured to the remaining meniscal rim

Fig. 2

A          B          C

FIG.3

A    B    C    D

EP 4 209 232 A1

**Description**

The invention relates to a new a collagen device for meniscus regeneration comprising a resilient resorbable chemically crosslinked collagen sponge wherein the external surface of the resilient resorbable chemically crosslinked collagen sponge is partially or totally covered by a dense layer of collagen fibers that is impermeable to meniscal cells, said collagen device for meniscus regeneration having an onset temperature of 40 to 70 °C and showing by mercury intrusion porosimetry interconnected pores with at least 60 % porosity with a pore diameter more than 10 $\mu$m, a process for preparing that collagen device for meniscus regeneration and the use of the latter in the knee region as an implant into a tear or segmental defect of the meniscus apt to be sutured to the remaining meniscal rim.

The meniscus acts in the knee joint as a crucial stabilizer, a mechanism for force distribution, and a lubricant in the area of contact between the tibia and the femur. Without the meniscus, stress concentration occurs in the knee in conjunction with abnormal joint mechanics, and premature development of arthrosis.

In the prior art, treatment of injured or diseased menisci has generally been both by surgical repair and by excision. With excision, regeneration of meniscal tissue may occur. Additionally, it is known that meniscal fibrochondrocytes have the ability to migrate into a defect filled with a fibrin clot and form tissue apparently similar to normal meniscal fibrocartilage. When an adequate matrix scaffold is present within a meniscal defect, such meniscalfibrocartilage may be formed. Meniscal tissue is also capable of self-repair when exposed to bleeding tissues, and additionally, it is also known in the prior art that meniscal cells in otherwise healthy joints are capable of cell division and matrix synthesis. Replacement of an injured meniscus in an otherwise healthy joint may prevent arthritic changes and may stabilize the joint. In diseased joints, replacement of the meniscus may reduce the progression of the disease process, and may provide pain relief.

Allografting or meniscal transplantation has been only partially successful over the long term due to the host's immunological response to the graft, failures in the cryopreservation process and failures of the attachment sites.

Protheses composed of artificial materials minimize the possibility of an immunological response. Generally, the replacement of meniscal tissue with structures consisting of artificial materials has been unsuccessful, principally because the opposing articular cartilage of animal joints is fragile and may not withstand abrasive forces. Additionally, known meniscus protheses of artificial materials have not been soft, durable or lubricative enough to withstand the high routine forces on the joints which are multiple of body weights.

EP-0324852-B1 discloses a prosthetic meniscus comprising a dry porous biocompatible bioresorbable fibers of a collagen wherein the fiber matrix is adapted to have an in vivo an outer surface contour substantially the same as that of a meniscus and comprising a three-dimensional array of type I collagen fibers interspersed with glycocosaminoglycan molecules, wherein said collagen fibers are present at a concentration of 65%-98% by dry weight, said glycocosaminoglycan molecules are present at a concentration 1%-25 % by dry weight, and provide crosslinks between said collagen fibers.

US-5,007,934 discloses a prosthetic meniscus comprising a dry porous biocompatible bioresorbable matrix of fibers of a collagen wherein the fiber matrix is adapted to have an in vivo an outer surface contour substantially the same as that of a meniscus, has a pore size from 50 to 500 $\mu$m, and establishes a partially bioresorbable scaffold adapted to ingrowth of meniscal fibrochondrocytes, and wherein said scaffold and said ingrown meniscal fibrochondrocytes support natural meniscal load forces.

US-6,042,610 discloses a meniscal augmentation device for implantation into a segmental defect of a meniscus that is a tear, wherein the device when implanted into the segmental defect establishes a bioresorbable scaffold of collagen fibers comprising a matrix having a pore size of from 50 to 500 $\mu$m adapted to the ingrowth of meniscal fibrochondrocytes, the scaffold and the ingrown meniscal chondrocytes support natural meniscal load forces and have a shape complementing the meniscal tear such that the in vivo outer surface of the composite of said meniscus and said device is substantially the same as that of a natural meniscus without segmental defects.

When implanted the prosthetic meniscus of EP-0324852-B1 or US-5,007,934 or the device for implantation into a meniscal tear of US-6,042,610 cannot be fixated or sutured sufficiently in all cases to the patient's meniscus due to its too fragile collagen matrix.

EP-3'055'000-B1 discloses a resilient resorbable chemically crosslinked collagen sponge for promoting soft tissue volume augmentation in the oral region, comprising 60-96 % (w/w) collagen and 4-40 % (w/w) elastin, which shows by mercury intrusion porosimetry interconnected pores with a median pore diameter between 50 and 90 $\mu$m and at least 80 % porosity with a pore diameter more than 10 $\mu$m, an onset temperature of 45 to 57 °C and a density in dry state from 50 to 65 mg/cm$^3$. That resilient resorbable chemically crosslinked collagen sponge is available under the name Geistlich Fibro-Gide® from Geistlich Pharma AG, Switzerland.

US-5,837,278 discloses a dense membrane of native collagen fibers extracted from pig peritoneum by a soft process preserving its natural properties. That dense membrane of native collagen fibers, available from Geistlich Pharma AG, Switzerland under the trademark Chondro-Gide®, has been used arthroscopically for cartilage and meniscus regeneration in the knee using e.g. the AMIC or AMMR technique (see Steinwachs, M. R., et al. (2019). "Systematic Review and Meta-Analysis of the Clinical Evidence on the Use of Autologous Matrix-Induced Chondrogenesis in the Knee." Cartilage: 1947603519870846and Ciemniewska-Gorzela, K., et al. (2020). "Complex Meniscus Tears Treated with Collagen Matrix Wrapping and Bone Marrow Blood Injection: Clinical Effectiveness and Survivorship after a Minimum of 5 Years' Follow-Up." Cartilage: 1947603520924762., respectively). That dense membrane of native collagen fibers become successfully sutured in the AMIC and AMMR technique.

The objective or problem of the invention is to find a device for meniscus regeneration that does not have the drawbacks of such devices of the prior art.

That problem is solved by the invention as defined in the claims.

The invention provides a collagen device for meniscus regeneration comprising a resilient resorbable chemically crosslinked collagen sponge wherein the external surface of the resilient resorbable chemically crosslinked collagen sponge is partially or totally covered by a dense layer of collagen fibers that is an impermeable to meniscal cells, said collagen device for meniscus regeneration having an onset temperature of 40 to 70 °C and showing by mercury intrusion porosimetry interconnected pores with at least 60 % porosity with a pore diameter more than 10 $\mu$m.

The term "impermeable to meniscal cells dense layer of collagen fibers" means here that the dense layer of collagen fibers has a barrier function as regards the meniscal cells, i.e. the cells residing in the vicinity of the meniscus, notably meniscal fibrochondrocytes, progenitor cells or fibroblast like cells (see Wang, J., et al. (2020). "Characterization of regional meniscal cell and chondrocyte phenotypes and chondrogenic differentiation with histological analysis in osteoarthritic donor-matched tissues." Sci Rep 10(1): 21658.). The meniscal cells thus cannot through the dense layer of collagen fibers until that dense layer of collagen fibers is sufficiently resorbed.

Generally, the dense layer of collagen fibers that is impermeable to meniscal cells has a density as measured by mercury intrusion porosimetry of 250 to 500 mg/cm$^3$ and the resilient resorbable chemically crosslinked collagen sponge has a density as measured by mercury intrusion porosimetry from 50 to 250 mg/cm$^3$.

The dense layer of collagen fibers allows sutureability of the collagen device to the remaining meniscal rim. By having a higher density of collagen of the dense layer of collagen fibers, sutureability of the collagen device is improved, as measured by the pull-out value (see Examples 7 and 8 ).

The dense layer of collagen fibers that is impermeable to meniscal cells may be an extracted natural membrane, preferably one which has been purified by a soft process preserving the collagen nativity. Such a soft process is disclosed in US-5,837,278.

A suitable such extracted natural membrane is Geistlich Chondro-Gide® whereby the collagen fibers have a high degree of nativity available from Geistlich Pharma AG, Switzerland..

Preferably, dense layer of collagen fibers that is impermeable to meniscal cells is an extracted natural membrane with collagen fibers having a high degree of nativity.

The term "high degree of nativity" means that the collagen shows at least 85 % native collagen fibers as determined after trypsin digestion as described in detail in Example 8 below

Another suitable extracted natural membrane is Alloderm® which was purified from skin (see US-5,336,616), in particular human skin.

That extracted natural membrane may be punctured with holes of 0.05 to 3.0 mm permeable to meniscal cells while maintaining enough sutureability.

The meniscal cell impermeable dense layer of collagen fibers may also be a reconstituted collagen membrane where individual collagen fibers, fibrils or molecular collagen (e.g. purified from tendons) or pieces of collagen tissues are reconstituted by e.g. compression and/or subsequent dehydration such to fulfill the above described requirements regarding cell impermeability, with possible chemical crosslinking. One method to obtain such a collagen layer is to arrange collagen fibers in close proximity to each other leading to pores of less than 10 $\mu$m in between. Such dense layers of collagen fibers are e.g. available from various companies under trade names or trademarks such as BioMend®, ACE-RCM6, Cytoplast RTM Collagen, GenDerm, OsseoGuard®, or Ossix® Volumax .

When the external surface of the resilient resorbable chemically crosslinked collagen sponge is totally covered by a dense layer of collagen fibers that is impermeable to meniscal cells, that dense layer of collagen fibers is punctured, e.g. with holes of 0.05 to 3.0 mm, such as to allow meniscal cells to enter the device, go into the interconnected pores and regenerate tissues.

Preferably, the external surface of the resilient resorbable chemically crosslinked collagen sponge is only partially, e.g. 10 to 90 %, covered by a dense layer of collagen fibers that is an impermeable to meniscal cells: The meniscal cells then can enter the device through the uncovered part of the external surface of the resilient resorbable chemically crosslinked collagen sponge, go into the interconnected pores and regenerate tissues.

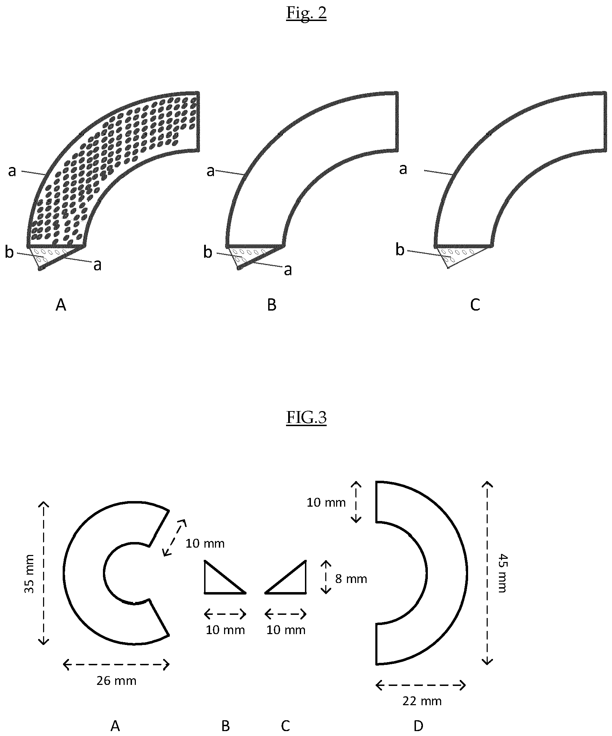

In one interesting embodiment (B in Figure 2), the external surface of the resilient resorbable chemically crosslinked collagen sponge is 50 to 90 % covered by a dense layer of collagen fibers that is an impermeable to meniscal cells.

In another interesting embodiment (A in Figure 2), the external surface of the resilient resorbable chemically crosslinked collagen sponge is 50 to 90 % covered by a dense layer of collagen fibers that is an impermeable to meniscal cells, that dense layer of collagen fibers being punctured with holes of 0.05 to 3.0 mm while maintaining sutureability.

In another interesting embodiment (C in Figure 2), the external surface of the resilient resorbable chemically crosslinked collagen sponge is 30 to 50 % covered by a dense layer of collagen fibers that is an impermeable to meniscal cells.

According to one embodiment the resilient resorbable chemically crosslinked collagen sponge includes both short and long collagen fibers, the presence of long collagen fibers increasing the tensional stability of the sponge part of the device. The long fibers containing sponges indeed show less sponge rupture upon simulated use in hydrated state in bench tests, or in surgical handling as shown by assays on cadavers (See Example 9).

Preferably, the collagen device for meniscus regeneration has been crosslinked using a crosslinking agent selected from the group consisting of gluteraldehyde, EDC (1-ethyl-3-(3-dimethylaminopropyl)-carbodiimide) and a mixture of EDC and NHS (N-hydroxysuccinimide).

Preferably, the collagen device for meniscus regeneration has been crosslinked in the presence of denaturized collagen fibers such as to increase the adherence of the resilient resorbable chemically crosslinked collagen sponge to the dense layer of collagen fibers that is an impermeable to meniscal cells.

The collagen device for meniscus regeneration shows by mercury intrusion porosimetry interconnected pores with at least 60 % porosity with a pore diameter more than 10 $\mu$m. Those interconnected pores having a diameter more than 10 $\mu$m allow invasion of meniscal cells into the collagen device such as to regenerate meniscal tissues.

Preferably, the collagen device for meniscus regeneration shows by mercury intrusion porosimetry at least 35 % porosity with a pore diameter between 30 and 300 $\mu$m.

Angiogenesis and tissue formation in devices with pore sizes from 30 to 300 $\mu$m is optimal as taught by van Tienen, T. G., et al. (2002). "Tissue ingrowth and degradation of two biodegradable porous polymers with different porosities and pore sizes." Biomaterials 23(8): 1731-1738, and Zhang, Z. Z., et al. (2016). "Role of scaffold mean pore size in meniscus regeneration." Acta Biomater 43: 314-326.

The term "resilient" here means that the chemically crosslinked collagen sponge part of the collagen device for meniscus regeneration is resistant to pressure, i.e. capable of regaining a large part of its original volume after being submitted to pressure *in vitro* or *in vivo*.

The resilient resorbable chemically crosslinked collagen sponge part of the collagen device for meniscus regeneration is crosslinked in presence of the dense layer of collagen fibers that is an impermeable to meniscal cells, under conditions close to those used for preparing Geistlich Fibro-Gide as described in EP-3'055'000-B1, in particular Example 5, and thus has similar resilience properties as the resorbable chemically crosslinked collagen sponge Geistlich Fibro-Gide; those conditions confer to the whole collagen device for meniscus regeneration similar resilience properties as Geistlich Fibro-Gide, as shown by cyclic compression tests: The collagen device for meniscus regeneration of the invention can thus act as a scaffold where new meniscus like tissue can be formed and then serve like the natural meniscus itself as a crucial stabilizer, a mechanism of distribution of high forces which may be multiple of body weights.

EP-3'055'000-B1 discloses that this resilience of the resorbable crosslinked collagen sponge requires an onset protein denaturation temperature of 45 to 57 °C and a density in dry state of that resorbable crosslinked collagen sponge of 50 to 65 mg/cm$^3$ and a pore size between 50 and 90 $\mu$m.

The onset temperature of protein denaturation is an important parameter for characterizing crosslinking of collagen that can be measured by Differentiation Scanning Calorimetry.

The onset temperature of protein denaturation should not exceed 70 °C since above this temperature wound healing complications may occur (see Delgado, L. M., et al. (2015). "To cross-link or not to cross-link? Cross-linking associated foreign body response of collagen-based devices." Tissue Eng Part B Rev 21(3): 298-313.)

Boekema B.K.L.H. et al., 2014, Journal of Material Sciences: Materials in Medicine, February 2014 25: 423-433, describe the effect on wound healing of pore size and crosslinking on collagen-elastin scaffolds used as dermal substitutes. The disclosed EDC-NHS chemically crosslinked collagen scaffolds contain 10-15 % elastin, have a pore size of 80 to 120 $\mu$m and show an onset temperature of 64 to 69 °C (see Table 1, page 425).

It has now been found that interesting resilience properties of the whole collagen device for meniscus regeneration can be achieved when the resorbable crosslinked collagen sponge has an onset temperature from 40 to 70 °C.

Preferably, the resilient resorbable chemically crosslinked collagen device has an onset temperature of 40 to 65 °C, in particular of 40 to 55 °C.

All prepared prototypes of the collagen device for meniscus regeneration wherein the resorbable chemically crosslinked collagen sponge has an onset temperature of 40 to 55 °C have shown in cyclic compressions tests, a retention of more than 70 % of the initial thickness and less than 55 % of the hysteresis in respect of the first loading after 49 cycles of compression to a pressure of 12.1 kPa in PBS at 37 °C.

Prepared prototypes of the collagen device for meniscus regeneration wherein the resorbable chemically crosslinked collagen sponge has an onset temperature of 55 to 65 °C have also shown interesting resilience properties in cyclic compressions tests interesting resilience properties.

The invention also concerns a process for preparing the above defined collagen device for meniscus regeneration

That collagen device for meniscus regeneration may be prepared by a process comprising:

(a) Preparing a dense layer of collagen fibers that is impermeable to meniscal cells by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, that dense layer of collagen fibers having a fibrous side allowing cell invasion and a smooth side acting as a barrier to meniscal cells, and cutting parts of dense layer of collagen fibers that is impermeable to meniscal cells

(b) Preparing a slurry of collagen fibers derived from porcine dermis by submitting grinded porcine dermis to a dehydrating treatment with a water soluble organic solvent, a degreasing treatment with an organic solvent, a basic treatment in a strong inorganic base at a pH above 12, an acid treatment in a strong inorganic acid at a pH from 0 to 1, rinsing with water and suspending the collagen fibers, freeze-drying, cleaning the dried collagen fibers with an organic solvent and grinding the cleaned dried fibers in an acidified solution of pH from 3 to 4,

(c) Preparing a slurry of short collagen fibers by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, milling the dry membrane obtained with a cutting mill, sieving through a 0.5-2 mm sieve and suspending the powder obtained in an acidified water solution of pH from 2.5 to 3.5,

(d) Preparing a slurry of long collagen fibers by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, milling the pieces of the hydrated membrane obtained with a mill comprising a serrated knife such as to obtain long collagen fibers,

(e) Preparing a collagen slurry mixture by mixing:

- 15 - 35 parts of the slurry of porcine dermis derived collagen fibers obtained in (b)
- 10 - 80 parts of the slurry of short collagen fibers derived from porcine or bovine peritoneum or pericardium obtained in (c) and optionally
- 0-60 parts of the slurry of long collagen fibers derived from porcine or bovine peritoneum or pericardium obtained in (d)peritoneum or pericardium obtained in (d).

(f) Assembling in a mold the collagen slurry mixture obtained in (e) and the cut parts of the dense layer of collagen fibers prepared in (a), the rough side facing the slurry, either on one side or on two sides of the slurry mixture, the rough side of the dense layer of collagen fibers facing the slurry, such as to obtain a coverage of the slurry mixture corresponding to the desired coverage of the sponge part after crosslinking,

(g) Freeze-drying the resulting assembly obtained in (f) and crosslinking the freeze-dried product in a solution containing a crosslinking agent, washing with water and freeze-drying, and

(h) Optionally sterilizing the freeze-dried product obtained in (f) by ethylene oxide sterilization, γ irradiation or X-ray irradiation.

According to an embodiment that process further comprises:

- (a1) Preparing a slurry of denaturized collagen fibers derived from porcine peritoneum by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, milling the dry membrane obtained with a cutting mill, sieving through a 0.5-2 mm sieve, acidifying the resulting collagen fibers in a slurry with repeated high pressure homogenization, and
- Adding to the slurry mixture prepared in (f), 2 - 10 parts of the slurry prepared in (a1).

Preferably in that process the crosslinking agent used in step (g) is selected from the group consisting of gluteraldehyde, EDC (1-ethyl-3-(3-dimethylaminopropyl)-carbodiimide) and a mixture of EDC and NHS (N-hydoxysuccinimide).

The invention also concerns the use of the collagen device for meniscus regeneration in the knee region as an implant into a tear or segmental defect of the meniscus apt to be sutured to the remaining meniscal rim.

BRIEF DESCRIPTION OF THE DRAWINGS

The invention will be described in further detail hereinafter with reference to illustrative examples of preferred embodiments of the invention and the accompanying drawings, in which:

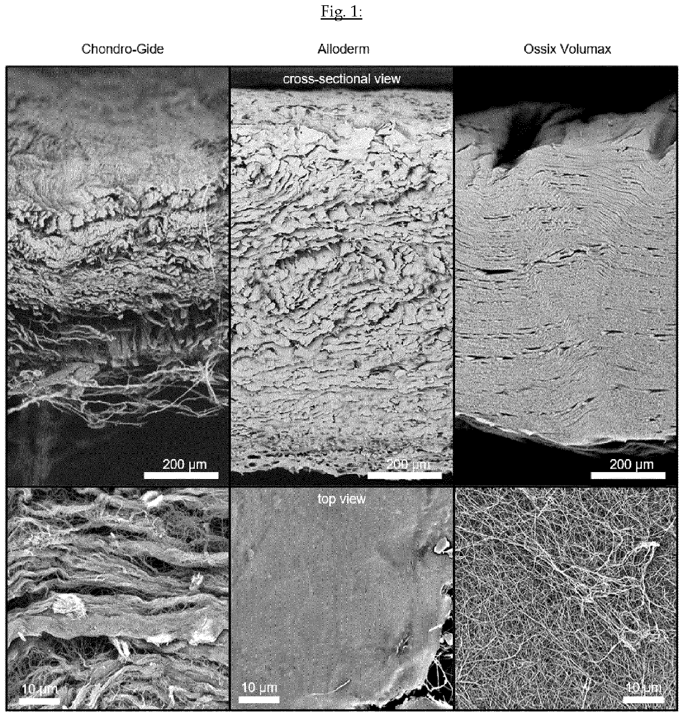

FIG.1 shows scanning electron micrographs SEMs of three commercially available dense layers of collagen fibers that are impermeable to meniscal cells, namely Geistlich Chondro-Gide®, Alloderm® and Ossix Volumax,

in cross-sectional view (upper row, bar 200 μm) and in top view (lower row, bar 10 μm). Geistlich Chondro-Gide@ (left, the fibrous side being visible in top view) is a purified bilayer collagen membrane derived for porcine peritoneum having a smooth side and a fibrous side with an average porosity of 70 - 80 % and the majority of pores between 0.1 - 40 μm; Alloderm® (middle) is a monolayer membrane purified from human skin with an approximate porosity of 60 % and the majority of pores between 0.1 - 10 μm; and
Ossix Volumax (right) is a reconstituted monolayer membrane with an approximate porosity of 90 % and the majority of pores between 0.1 - 10 μm.

FIG.2 represents schematic drawings of three preferred embodiments A, B and C of the device for meniscus regeneration of the invention.

In embodiments A and B, the dense layer of native collagen fibers (a, bold) is placed proximally and distally (A and B), thus covering about 50-90 % of the external surface of the resilient collagen sponge.
In embodiment B, the dense layer of native collagen fibers (a, bold) is intact.
In embodiment A, the dense layer of native collagen fibers (a, bold) has been punctured with holes of 0.05 to 3.0 mm to increase cell permeability.
In embodiment C, the dense layer of native collagen fibers (a, bold) is placed only proximally, thus covering 30 - 50 % of the external surface of the resilient collagen sponge.
The inner volume and the face towards the patient meniscus consist of a collagen sponge comprising interconnected pores >30 μm (b).
FIG. 3 represents possible sizes of the collagen device for meniscus regeneration for a lateral meniscus (A, B) and a medial meniscus (C, D), from the top (A: 26 X 35 X 10 mm, D: 22 X 45 X 10 mm) and in cross-sections (B, C : 8 X 10 mm).
FiG. 4 shows the typical result of a cyclic compression test of a resilient resorbable chemically crosslinked collagen sponge obtained in Example 6
FiG. 5 shows the cyclic compression tests of the device for meniscus regeneration of the invention, for the prototypes of Examples 7.4 and 7.5.
The following examples illustrate the invention without limiting its scope.

**EXAMPLE 1** Preparation of the dense layer of native collagen fibers

As described in "Example" of US-5,837,278, peritoneal membranes from young pigs were completely freed from flesh and grease by mechanical means, washed under running water and treated with 2% NaOH solution for 12 hours. The membranes were then washed under running water and acidified with 0.5% HCl. After the material had been acidified through its entire thickness (for about 15 minutes) the material was washed with water until a pH of 3.5 was obtained. The material was then shrunk with 7% saline solution, neutralised with 1% NaHCO$_3$ solution and washed under running water. The material was then dehydrated with acetone and degreased with n-hexane and dried using ethanol ether.
A dense extracted natural membrane of native collagen fibers was thus obtained.
Alternatively, a commercial Geistlich Chondro-Gide@ membrane was used for the dense layer of native collagen fibers.
Both membranes are impermeable to meniscal cells.
As shown by scanning electron microscopy, the smooth/upper layer consists of densely arranged collagen fibers. The distance between the collagen fibers is substantially lower than 10 μm (as seen in Figure 1). In addition, mercury intrusion porosimetry of both membranes show that the majority of pores are between 0.1- 40 μm.

**EXAMPLE 2** Preparation of a slurry of collagen fibers derived from pig dermis.

Porcine hides were ground in a meat grinder to pieces of 1 to 20 mm. The water was removed using a water soluble solvent such as an alcohol or a ketone. The collagen fibers were defatted using a chlorinated hydrocarbon such as dichloroethane or methylene chloride or a non-chlorinated hydrocarbon such as hexane or toluene. After removing the solvent the collagen was treated with a strong inorganic base at a pH above 12 for a period of 6 to 24 hours and treated with a strong inorganic acid at a pH of 0 to 1 for a period of 1 to 12 hours. The excess acid was removed by rinsing with water and the suspension was homogenized by colloid milling to a 0.5 to 2 % homogenous suspension of collagen fibers in the presence of a swelling regulator such as an inorganic salt. The suspension was dried by freeze-drying and the dry collagen fibers were successively cleaned with different organic solvents such as alcohols, ethers, ketones and chlorinated hydrocarbons, the solvents being then evaporated under vacuum to a solvent residue of less than 1 % (w/w).

A 3.75 % (w/w) collagen slurry of collagen fibers were prepared by finely grinding by colloid milling adequate amounts of the cleaned dry fibers obtained above with water at a pH of 3.4.

**EXAMPLE 3** Preparation of a slurry of short collagen fibers derived from porcine peritoneum

A dense porcine peritoneum membrane of native collagen fibers extracted and purified as described in paragraph 1 of Example 1, or alternatively a Chondro-Gide@ membrane (available from Geistlich Pharma AG, CH-6110, Switzerland), was cut using scissors and milled with a cutting mill which includes a trapezoidal sieve of 0.5 to 1.0 mm, thus giving short collagen fibers. A 4 % (w/w) collagen slurry of short collagen fibers and a 6 % (w/w) collagen slurry of short collagen fibers were prepared by suspending adequate amounts of the dried powder in water and adjusting the pH to 2.6 with 10 mM hydrochloric acid.

**EXAMPLE 4** Preparation of a slurry of long collagen fibers derived from porcine peritoneum or bovine tendons

A dense porcine peritoneum membrane of native collagen fibers extracted and purified as described in paragraph 1 of Example 1, or alternatively a Chondro-Gide@ membrane (available from Geistlich Pharma AG, CH-6110, Switzerland), was cut using scissors into pieces of several centimetres. Then the collagen pieces were mixed with water to a final concentration of 5.5 %. The pH was adjusted to pH 5.0 using phosphoric acid and placed in a mill comprising a serrated knife (Rotor Lips AG, robot coupe 4.4). The peritoneum was cut into long collagen fibers at low temperatures by applying multiple cycles of 1 min duration at a speed of 3'500 rpm. A slurry of long collagen fibers derived from a porcine peritoneum was thus obtained.

An alternative way to obtain a slurry of long collagen fibers was to buy purified bovine tendon collagen fibers from commercial vendors. Then they were hydrated in water and the pH was adjusted towards acidic such to obtain a swollen collagen slurry. A slurry of long collagen fibers derived from tendons was thus obtained.

These long collagen fibers are used to increase tensional stability of the sponge part of the device for meniscus regeneration. Long collagen fibers are substantially longer than 1 mm and can be as long as several centimetres, as determined by visual examination.

**EXAMPLE 5** Preparation of a slurry of denaturized collagen fibers derived from a porcine peritoneum

A dense porcine peritoneum membrane of native collagen fibers extracted and purified as described in paragraph 1 of Example 1, or alternatively a Chondro-Gide@ membrane (available from Geistlich Pharma AG, CH-6110, Switzerland), was cut using scissors into pieces of several centimetres and milled with a cutting mill (e.g. Pulverisette 25 from Fritsch: see www.fritsch.de./produkte/mahlen/schneidmuehlen/pulverisette-25 or SM300 from Retsch: www.retsch.de/de/produkte/zerkleinern/schneidmuehlen.htlm) which includes a trapezoidal sieve of 0.5 to 1 .0 mm. The resulting collagen fibers are hydrated in an aqueous $H_3PO_4$ solution of pH 3.5 at a concentration of 3 % and denaturized using a high pressure homogenizer at 1500-2000 bar, that mixing being repeated several times. In a next step, the pH is neutralized to pH 7.0 by adding a sodium hydroxide solution and diluted to a final concentration of 2.5 %.

A slurry of denaturized collagen fibers derived from a porcine peritoneum was thus obtained.

**EXAMPLE 6** Preparation of a resilient resorbable collagen sponge chemically crosslinked with a mixture of EDC and NHS (as in Example 5 of EP3'055'000-B1)

Different ratios of the collagen preparation were mixed in various ratios and tested towards mechanical resilience und compression, tension and suture pull-out.

Four parts of the 6 % (w/w) slurry of short collagen fibers obtained in Example 3 were mixed with one part of the 3.75 % (w/w) slurry of collagen fibers obtained in Example 2 and poured into molds. The resulting 5.55 % slurry of collagen fibers was dried by freeze-drying at - 10 °C. These sponges were chemically crosslinked in a solution containing 0.2 M MES (2-(N-morpholino)-ethanesulfonic acid) and 50% w/w ethanol at a pH of 5.5 with 0.1g EDC (1-ethyl-3-(3-dimethylaminopropyl)-carbodiimide) per gram collagen and 0.1 g NHS (N-hydoxysuccinimide) per gram collagen, under agitation at room temperature for a period of 120 min. The chemically crosslinked sponges were washed first with 0.1 M $Na_2HPO_4$ solution, 1M NaCl solution, 2M NaCl solution, then with water and freeze dried at -10 °C.

The dried chemically crosslinked collagen sponges were sterilized by irradiation at e.g. 26 kGy. The measured onset temperature and density in dry state of the sterilized chemically crosslinked collagen sponges were 45 - 57 °C and 50 - 56 mg/cm³, respectively.

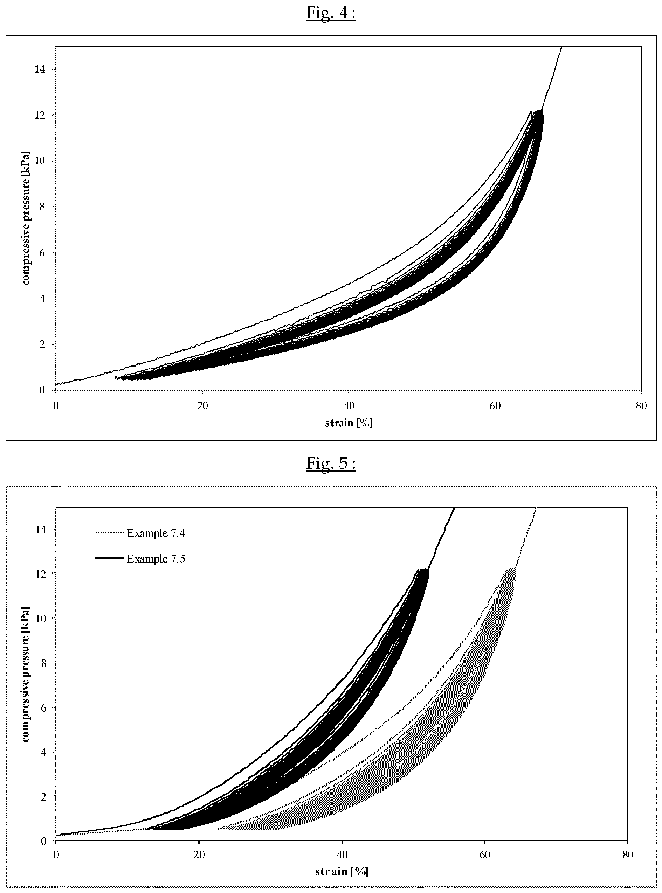

FiG. 4 shows the typical result of a cyclic compression test of a resilient resorbable chemically crosslinked collagen sponge : After 49 cycles of compression to a pressure of 12.1 kPa in PBS at 37 °C, the sterilised chemically crosslinked collagen sponges showed a retention of over 70 % of its initial thickness and less than 55 % of the hysteresis in respect of the first loading.

Mercury intrusion porosimetry showed for the sterilised chemically crosslinked collagen sponges a median pore diameter of 70 $\mu$m and more than 80 % porosity with a pore diameter more than 10 $\mu$m.

Since this prototype is not mechanically reinforced by a dense collagen layer, the suture pull-out values are low: an average of about 0.2 N, as determined on 6 samples by the test described in Example 8.

**EXAMPLE 7** Preparation of different prototypes of the device for meniscus regeneration of the invention chemically crosslinked with a mixture of EDC and NHS.

Mixtures of different ratios of the slurries of collagen fibers obtained in Examples 2 and 3, and optionally, the slurry of long collagen fibers derived from porcine peritoneum or bovine tendons of Example 4, and /or the denaturated collagen slurry derived from porcine peritoneum of Example 5 , were used and chemically crosslinked with a mixture of EDC and NHC using appropriate mold s in presence of the dense membrane of collagen fibers obtained in Example 1, either on one side or on two sides of the mixture of slurries, the rough side of the collagen membrane facing the slurry.

The prototypes of the device for meniscus regeneration of the invention thus obtained were analytically characterized using the methods and protocols described in Example 8.

Mold s used to obtain from the prepared collagen slurry and the dense layer of collagen fibers were made for instance from stainless steel, PEEK, aluminium or any other suitable material. It is a plate containing multiple recesses in the shape and with the dimensions as shown in Figure 3. With each plate several prototypes were prepared in one run.

For each of the examples 7.1 to 7.7, a dense layer/membrane of collagen fibers obtained in Example 1 was cut to the desired size. Then the collagen slurry was mixed using at least two of the different starting materials as described in Examples 2 - 5. The dense collagen fibers layer and the collagen slurry were then assembled in the molds such that the dense collagen fibers layer was placed as shown on the embodiments shown in Figure 2, the rough side of the membrane facing the slurry mixture.

- As described in example 7.8, for all embodiments of examples 7.1 - 7.7, the in the molds prepared samples, were subjected to freeze drying. During freeze drying, the pore size was determined. Then samples were cross-linked and washed. After that, samples were dried by freeze drying or vacuum drying after immersion in volatile solvents. Then samples of the full device were packed and sterilized by x-ray irradiation at e.g. 26 kGy, or by ethylene oxide.

- EXAMPLE 7.1: Collagen device for meniscus regeneration wherein the resilient resorbable crosslinked collagen sponge is covered on both sides by a dense layer of collagen fibers that is impermeable to meniscal cells (Embodiment B of Fig. 2) Dense layers of native collagen fibers, the membranes obtained in Example 1, were cut to the require sizes to confirm to the desired external surfaces and hydrated in the slurry mixture prepared in Example 6, namely 75 parts of the 6 % (w/w) slurry of short collagen fibers obtained in Example 3 and 25 parts of the 3.75 % (w/w) slurry of collagen fibers obtained in Example 2.
  In that embodiment, cut layers of the dense layer of collagen fibers were placed in molds and the slurry mixture was added, the dense layers covering approximately 50

    - 90 % of the external surface of the slurry mixture.

As described in Example 7.8, those assemblies were then used to build the final sterilized device.

- EXAMPLE 7.2: Collagen device for meniscus regeneration wherein the resilient resorbable crosslinked collagen sponge is covered on both sides by a dense layer of collagen fibers that is punctured (Embodiment A of Fig. 2) In that embodiment, the same steps as in Example 7.1 were used, the only difference being that the dense layer of native collagen fibers was punctured with holes of 0.05 - 3 mm, e.g. using a needle band containing 50 needles per cm² with a shaft diameter of 0.88 mm.

The punctured dense layer of native collagen fibers was then used to build the final sterilized device as described in Example 7.8.

- EXAMPLE 7.3: Collagen device for meniscus regeneration wherein the resilient resorbable crosslinked collagen sponge is covered on one side by a dense layer of collagen fibers that is impermeable to meniscal cells (Embodiment C of Fig. 2) Dense layers of native collagen fibers, the membranes obtained in Example 1, were cut to the required sizes to confirm to the desired external surfaces and hydrated in a slurry mixture similar to that prepared in Example 6, namely 75 parts of the 6 % (w/w) slurry of short collagen fibers obtained in Example 3 and 25 parts of the 3.75 % (w/w) slurry of collagen fibers obtained in Example 2.

In that embodiment, the slurry mixture was added and cut layers of the dense layer of collagen fibers were placed in molds, the dense layers covering approximately 30 - 50 % of the external surface of the slurry mixture.

As described in Example 7.8, those assemblies were then used to build the final sterilized device.

- EXAMPLE 7.4: Collagen device for meniscus regeneration wherein the resilient resorbable crosslinked collagen sponge comprising long fibers is covered on both sides by a dense layer of collagen fibers that is impermeable to meniscal cells

(Embodiment B of Fig. 2).

The membranes/dense layers of collagen fibers obtained in Example 1, were cut to the required sizes to confirm to the desired external surfaces and hydrated in a mixture slurry consisting of 63.8 parts of the 6 % (w/w) slurry of short collagen fibers obtained in Example 3, 25 parts of the 3.75 % (w/w) slurry of collagen fibers obtained in Example 2 and 11.3 parts of the 5.5 % (w/w) slurry of long collagen fibers derived from porcine peritoneum obtained in Example 4.

In that embodiment, the slurry mixture was added and cut layers of the dense layer of native collagen fibers were placed in molds, the dense layers covering approximately 50 - 90 % of the external surface of the slurry mixture.

As described in Example 7.8, those assemblies were then used to build the final sterilized device. Long collagen fibers were here added to increase the tensional stability of the sponge part.

- EXAMPLE 7.5: Collagen device for meniscus regeneration wherein the resilient resorbable crosslinked collagen sponge comprising long fibers derived from porcine peritoneum and denaturized collagen fibers is covered on both sides by a dense layer of collagen fibers that is impermeable to meniscal cells (Embodiment B of Fig. 2) Dense layers of native collagen fibers, the membranes obtained in Example 1, were cut to the require sizes to confirm to the desired external surfaces and hydrated in a slurry mixture comprising 25 parts of the 3.75 % (w/w) slurry of collagen fibers obtained in Example 2, 56.3 parts of the 6 % (w/w) slurry of short collagen fibers obtained in Example 3, 11.3 parts of the 5.5 % (w/w) slurry of long collagen fibers derived from porcine peritoneum obtained in Example 4 and 7.5 parts of the 2.5 % (w/w) denaturized collagen fiberslurry obtained in Example 5.

In that embodiment, the slurry mixture was added and cut layers of dense layer of collagen fibers were placed in molds, the dense layers covering approximately 50 - 90 % of the external surface of the slurry mixture.

As described in Example 7.8, those assemblies were then used to build the final sterilized device. Long collagen fibers were here added to increase the tensional stability of the sponge part and denaturized collagen fibers were added to increase the adhesion of the dense collagen layer to the sponge part.

- EXAMPLE 7.6 Collagen device for meniscus regeneration wherein the resilient resorbable crosslinked collagen sponge comprising long fibers derived from bovine tendons and denaturized collagen fibers is covered on both sides by a dense layer of collagen fibers that is impermeable to meniscal cells

(Embodiment B of Fig. 2)

Dense layers of native collagen fibers, the membranes obtained in Example 1, were cut to the require sizes to confirm to the desired external surfaces and hydrated in a slurry mixture comprising 25 parts of the 3.75 % (w/w) slurry of collagen fibers obtained in Example 2, 15 parts of the 6 % (w/w) slurry of short collagen fibers obtained in Example 3 and 52.5 parts of the 5.5 % (w/w) slurry of long collagen fibers derived from bovine tendons obtained in Example 4 and 7.5 parts of the 2.5 % (w/w) denaturized collagen fiberslurry obtained in Example 5.

In that embodiment, the slurry mixture was added and cut layers of dense layer of native collagen fibers were placed in molds, the dense layers covering approximately 50 - 90 % of the external surface of the slurry mixture.

As described in Example 7.8, those assemblies were then used to build the final sterilized device.

- EXAMPLE 7.7: Collagen device for meniscus regeneration wherein the resilient resorbable crosslinked collagen sponge was prepared with a higher density and comprising long fibers derived from porcine peritoneum using denaturized collagen fibers is covered on both sides by a dense layer of collagen fibers that is impermeable to meniscal cells

(Embodiment B of Fig. 2)

Dense layers of native collagen fibers, the membranes obtained in Example 1, were cut to the require sizes to confirm to the desired external surfaces and hydrated in a slurry mixture comprising 25 parts of the 3.75 % (w/w) slurry of collagen fibers obtained in Example 2, 50.4 parts of the 6 % (w/w) slurry of short collagen fibers obtained in Example 3, 14.4 parts of the 5.5 % (w/w) slurry of long collagen fibers derived from porcine peritoneum obtained in Example 4 and 7.2 parts of the 2.5 % (w/w) denaturized collagen fiber slurry obtained in Example 5, to which 3 parts of dry powder of short collagen fibers described as intermediate product in Example 3 were added.

In that embodiment, the slurry mixture was added and cut layers of dense layer of native collagen fibers were placed in molds, the dense layers covering approximately 50 to 90 % of the external surface of the slurry mixture.

As described in Example 7.8, those assemblies were then used to build the final sterilized device.

In example (7.7), the overall density was increased.

- EXAMPLE 7.8: Cross-linking and sterilization of all embodiments as described in above Examples 7.1 - 7.7.

Then the assemblies of dense collagen fibers layers and the slurry mixtures in mold s corresponding to the final sizes of the collagen device for meniscus regeneration as described in Fig 3 were dried by freeze drying at - 10 °C. These assemblies were chemically crosslinked in a solution containing 0.2 M MES (2-(N-morpholino)-ethanesulfonic acid) and 50% w/w ethanol at a pH of 5.5 with 0.1g EDC (1-ethyl-3-(3-dimethylaminopropyl)-carbodiimide) per gram collagen and 0.1 g NHS (N-hydoxysuccinimide) per gram collagen, under agitation at room temperature for a period of 120 min. The chemically crosslinked sponges were washed first with 0.1 M $Na_2HPO_4$ solution, 1M NaCl solution, 2M NaCl solution, then with water and freeze dried at - 10 °C. Alternatively, prototypes can also become dried after immersion in solvents and drying in air or in a vacuum drier.

The dried and chemically crosslinked full collagen devices for meniscus regeneration were sterilized most of them by X-ray irradiation at 26 kGy and a few of them by ethylene oxide.

The measured onset temperatures of protein denaturation of the X-ray irradiation sterilized prototypes of the collagen device for meniscus regeneration were 40 - 55 °C. All those prototypes showed in cyclic compressions tests, a retention of more than 70 % of the initial thickness and less than 55 % of the hysteresis in respect of the first loading after 49 cycles of compression to a pressure of 12.1 kPa in PBS at 37 °C.

Higher onset temperatures of 55 to 65 °C were observed on the ethylene oxide sterilized prototypes of the collagen device for meniscus regeneration or on protypes where a higher concentration of the mixture of EDC and NHS crosslinker was used. Such prototypes also showed interesting resilience properties in cyclic compression tests.

Mercury intrusion porosimetry showed for the prepared prototypes of the collagen device for meniscus regeneration at least 60 % porosity of interconnected pores with a diameter more than 10 $\mu$m, at least 40 % porosity of interconnected pores with a diameter more than 30 $\mu$m, at least 35 % porosity of interconnected pores with a diameter from 30 to 300 $\mu$m, and at least 30 % porosity of interconnected pores with a diameter from 30 to 150 $\mu$m.

As expected, embodiments with lower density had higher porosity than embodiments with higher density. The pore size diameter could be set by adjusting freezing rates during freeze drying.

Because these prototypes were mechanically reinforced by a dense collagen layer, the suture pull-out values are high: about 2 - 6 N.

**EXAMPLE 8** Analytical characterization of the different prototypes of the device for meniscus regeneration of the invention

**Cyclic compression test**

The % of initial thickness and hysteresis in respect to the first loading after 49 cycles of compression to a pressure of 12.1 kPa were measured using a mechanical compression machine, namely Z2.5 material compression machine manufactured by Zwick Roell, using the program Test Expert II.

The measurements were performed submerged in PBS at 37 °C on sterile samples which had been incubated for 2 hours at 37°C in a PBS solution of pH 7.4 (prepared by dissolving 80.0 g NaCl, 2.0 g KCl, 17.7 g $Na_2HPO_4$ and 2.4 g $KH_2PO_4$ in 1000 ml water, diluting ten times the solution in water and adjusting the pH with HCl to 7.4).

Samples were subjected to a total of 49 cycles of loading between 0.5 and 12.1 kPa at a strain rate of 33 % of initial height per min, analysis starting at a pre-pressure of 0.25 kPa.

The initial height at 0.5 kPa pressure was used to calculate the retention of initial height after 49 cycles of loading and unloading. The "hysteresis in respect to the first loading" is the percentage work which was dissipated during unloading using the work (W$_1$, loading in Nm) between 0.5 and 12.1 kPa of the first loading and the work from the 49$^{th}$ unloading cycle (W$_{49}$, unloading in Nm)

according equation XY:

$$\text{Eq XY: "Hysteresis in respect to the first loading" [\%]} = \frac{100 * (W_{1,\,loading} - W_{49,\,unloading})}{W_{1,\,loading}}$$

The program Test Expert or Excel calculates the % of retention of initial thickness and the % of retention of initial hysteresis after 49 cycles of compression to a pressure of 12.1 kPa.

Figure 4 shows the typical result of a cyclic compression test.

**Onset of protein denaturation temperature determination by differential scanning calorimetry**

The resilient resorbable chemically crosslinked collagen samples were cut into pieces of e.g. 1 - 3 mg of collagen material and placed into an aluminium differential scanning calorimetry pan and hydrated in e.g. 40 µL of phosphate buffered saline pH 7.4 and closed. The measurement is done using a closed reference aluminium pan of the same size filled with e.g. 40 µL of phosphate buffered saline at 5 Kelvin per minute from e.g. 5 - 95 °C. The onset of protein denaturation temperature is determined for each sample. The onset temperature of protein denaturation shall not exceed 70 °C since above this temperature wound healing complications are foreseen (Delgado, L. M., et al. (2015). "To cross-link or not to cross-link? Cross-linking associated foreign body response of collagen-based devices." Tissue Eng Part B Rev 21(3): 298-313.). Since the device is chemically cross-linked and shall not degraded immediately after implantation, a minimal cross-linking temperature of 40 °C is reasonable.

**Mercury intrusion porosimetry**

Pore size distribution was measured on dry samples according ISO 15901-1 by mercury intrusion porosimetry. Briefly, samples cut to size suitable to become placed in a penetrometer were measured at low pressure. Data was evaluated using a contact angle ⵣ of 145° and a surface tension of mercury of $\gamma$=0.485 N m$^{-1}$) The bulk density was determined by mercury intrusion pycnometry and used for the calculation of the porosity.

**Determination of the % of native Collagen**

According to Ph. Eur. 2.2.29, USP <621> Liquid Chromatography:

*Principle:* The 4-hydroxyproline content of the sample material is determined by amino acid analysis after hydrolysis with hydrochloric acid (= total collagen content) and trypsin (= non-native collagen content). The percentage content of native collagen is calculated.

| | |
|---|---|
| Mobile phase A: | Buffer pH 6.4: 0.05 M sodium acetate in water. pH adjustment with acetic acid. |
| Mobile phase B: | Acetonitrile / Water = 80:20 |
| Diluent: | Water / Methanol = 1:1 |
| NaHCO$_3$-Buffer: | 0.1 N Sodium hydrogen carbonate solution in water |
| Trypsin-solution: | 1.8 mg/ml Trypsin in NaHCO$_3$-Buffer |
| Standard-Mix: | In a 245:35:10:10 mixture of methanol / water /trimethylamine / phenyl isothiocyanate the Standard-Mix is derivatisized in a concentration of 7.33 mg/Ml. Dilution of the resulting mixture by a factor of 50. |

Reference solution: 400 parts [µl] of an 80 µg/ml solution of 4-Hydroxyproline in water are evaporated to dryness in a speed-vac system at about 50°C. After derivatisation in 100 parts [µl] of a mixture of methanol / water / triethyl-amine / phenyl isothiocyanate (80:10:5:5) for about 60 min at about 60°C the resulting solution is diluted with 1900 parts

[μl] of diluent.

Sample solution 1: A suspension of 2 mg/ml sample material in 3M HCl is heated in a hydrolysis tube at about 105°C for about 15 hours. The resulting solution is diluted with water to a concentration of 0.8 mg/ml. 400 parts [μl] of this solution are evaporated to dryness in a speed-vac system at about 50°C. After derivatisation in 100 parts [μl] of a mixture of methanol / water / trimethylamine / phenyl isothiocyanate (80:10:5:5) for about 60 min. at about 60°C the resulting solution is diluted with 1900 parts [μl] of diluent.

Sample solution 2: 1 mg/ml sample material in a mixture of $NaHCO_3$-Buffer / Trypsin-solution 5:1 are agitated for about 6 hours at room temperature. After ultra-filtration 3000 parts [μl] are mixed with the same volume of 6M HCl and the solution is heated in a hydrolysis tube at about 105°C for about 15 hours. The resulting solution is diluted with water to get a concentration of 0.3 mg/ml (in respect to the sample material). 2000 parts [μl] of this solution are evaporated to dryness in a speed-vac system at about 50°C. After derivatisation in 100 parts [μl] of a mixture of methanol / water / trimethylamine / phenyl isothiocyanate (80:10:5:5) for about 60 min at about 60°C the resulting solution is diluted with 1400 parts [μl] of diluent.

| Column: | YMC-Pack ODS-AQ 15cm x 4.6cm, 3 μm or equivalent. |
|---|---|
| Flow: | 1.0 ml/min |
| Column temp.: | 45°C |
| Injection volume: | 10 μl |
| Detection: | UV at 254 nm |
| Run time: | 40 min |
| Gradient: | |

| Time [min] | Flow [ml/min] | % A | % B |
|---|---|---|---|
| Initial | 1.0 | 95 | 5 |
| 25 | 1.0 | 50 | 50 |
| 28 | 1.0 | 20 | 80 |
| 33 | 1.0 | 20 | 80 |
| 35 | 1.0 | 95 | 5 |
| 40 | 1.0 | 95 | 5 |

The amount of 4-Hydroxyproline in both solutions is calculated against the reference solution and the percentage content of native collagen. The amino acid composition is evaluated.

All amounts may be adjusted as long as the concentrations and ratios remain unchanged.

**Suture pull-out force determination**

To assess sutureability of the device to the remaining meniscal rim, a vertical suture using an ultra-braided size 2-0 suture is made, then the suture pull-out force is determined on an uniaxial tensile testing machine. The suture is placed on the proximal plane of the device 4 mm from the edge facing the remaining rim of the meniscus. Then the specimens are hydrated at 37 °C in phosphate buffered saline, pH 7.4, for 15 min, and mounted on the tensile testing machine. The suture pull-out force is determined at 30 mm per minute, submerged in phosphate buffered saline at 37 °C.

**EXAMPLE 9 Essays on human cadavers**

Prepared prototypes of the collagen device for meniscus regeneration were tested arthroscopically in human cadaver knee surgeries. A partial meniscectomy was prepared in the cadaver knee. The defect size was estimated using a measuring device. Then the dry material was cut to size and hydrated in Ringer solution. The device was clamped with forceps and placed arthroscopically into the partial meniscetomized defect. Due to the inherent mechanical resilient properties of the device, placement was easy without any breakage of the device. Then, vertical and horizontal sutures were placed using e.g. all-inside suturing technique or an inside-out suturing technique. Tight fixation was checked by pulling the device centrally using a hock. Good placement was assessed by bringing the knee from flexion to extension for three times. The tested devices all remained stable and in place. A higher stability of the sponge part of the device was observed for the prototypes where the sponge included both short and long collagen fiber s compared to prototypes where the sponge only included both short collagen fiber s.

**EXAMPLE 10 Animal assays**

Samples are tested in an ovine partial meniscectomy sheep model (see Lee, C. H., et al. (2014). "Protein-releasing polymeric scaffolds induce fibrochondrocytic differentiation of endogenous cells for knee meniscus regeneration in sheep." Sci Transl Med 6(266): 266ra171.). The device is sutured into surgically created defects and then let heal for 6 months. Osteoarthritis of the congruent cartilage can be assessed visually using the Outerbridge score and histological using the Mankin score. In addition, degradation of the device and neo-formation of meniscus tissue is assessed by standard histological investigation.

**Claims**

1.  A collagen device for meniscus regeneration comprising a resilient resorbable chemically crosslinked collagen sponge wherein the external surface of the resilient resorbable chemically crosslinked collagen sponge is partially or totally covered by a dense layer of collagen fiber s that is impermeable to meniscal cells, said collagen device for meniscus regeneration having an onset temperature of 40 to 70 °C and showing by mercury intrusion porosimetry interconnected pores with at least 60 % porosity with a pore diameter more than 10 $\mu$m.

2.  A collagen device according to claim 1 wherein the external surface of the resilient resorbable chemically crosslinked collagen sponge is 10 to 90 % covered by dense layer of collagen fibers that is impermeable to meniscal cells.

3.  A collagen device according to claim 2 wherein the external surface of the resilient resorbable chemically crosslinked collagen sponge is 50 to 90 % covered by a dense layer of collagen fibers that is impermeable to meniscal cells.

4.  A collagen device according to claim 2 wherein the external surface of the resilient resorbable chemically crosslinked collagen sponge is 30 to 50 % covered by a dense layer of collagen fibers that is impermeable to meniscal cells.

5.  A collagen device according to any of claims 1 to 4 wherein the dense layer of collagen fibers that is impermeable to meniscal cells is an extracted natural membrane.

6.  A collagen device according to claim 5 wherein the extracted natural membrane that is impermeable to meniscal cells is punctured with holes of 0.05 to 3.0 mm.

7.  A collagen device according to claim 5 or 6, wherein the extracted natural membrane shows a high degree of nativity.

8.  A collagen device according to any of claims 1 to 7, which shows by mercury intrusion porosimetry at least 35 % porosity with a pore diameter between 30 and 300 $\mu$m.

9.  A collagen device according to any of claims 1 to 8, wherein the resilient resorbable chemically crosslinked collagen sponge has an onset temperature of 40 to 65 °C.

10. A collagen device according to any of claims 1 to 9, wherein the resilient resorbable chemically crosslinked collagen sponge includes short and long collagen fibers.

11. A collagen device according to any of claims 1 to 10, which has been crosslinked using a crosslinking agent selected from the group consisting of gluteraldehyde, EDC (1-ethyl-3-(3-dimethylaminopropyl)-carbodiimide) and a mixture of EDC and NHS (N-hydoxysuccinimide).

12. A collagen device according to any of claims 1 to 11, which has been crosslinked in the presence of denaturized collagen fibers such as to increase the adherence of the resilient resorbable chemically crosslinked collagen sponge to the dense layer of collagen fibers that is an impermeable to meniscal cells.

13. A process for preparing the device of any of claims 1 to 12 comprising:

    (a) Preparing a dense layer of collagen fibers that is impermeable to meniscal cells by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, that dense layer of collagen fibers having a fibrous side allowing cell invasion and a smooth side acting as a barrier to meniscal cells, and cutting parts of dense layer of collagen fibers that is impermeable to meniscal cells

(b) Preparing a slurry of collagen fibers derived from porcine dermis by submitting grinded porcine dermis to a dehydrating treatment with a water soluble organic solvent, a degreasing treatment with an organic solvent, a basic treatment in a strong inorganic base at a pH above 12, an acid treatment in a strong inorganic acid at a pH from 0 to 1, rinsing with water and suspending the collagen fibers, freeze-drying, cleaning the dried collagen fibers with an organic solvent and grinding the cleaned dried fibers in an acidified solution of pH from 3 to 4,

(c) Preparing a slurry of short collagen fibers by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, milling the dry membrane obtained with a cutting mill, sieving through a 0.5-2 mm sieve and suspending the powder obtained in an acidified water solution of pH from 2.5 to 3.5,

(d) Preparing a slurry of long collagen fibers by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, milling the pieces of the hydrated membrane obtained with a mill comprising a serrated knife such as to obtain long collagen fibers,

(e) Preparing a collagen slurry mixture by mixing:

- 15 - 35 parts of the slurry of porcine dermis derived collagen fibers obtained in (b)
- 10 - 80 parts of the slurry of short collagen fibers derived from porcine or bovine peritoneum or pericardium obtained in (c) and optionally
- 0 - 60 parts of the slurry of long collagen fibers derived from porcine or bovine peritoneum or pericardium obtained in (d),

(f) Assembling in a mold the collagen slurry mixture obtained in (e) and the cut parts of the dense layer of collagen fibers prepared in (a), the rough side facing the slurry, either on one side or on two sides of the slurry mixture, the rough side of the dense layer of collagen fibers facing the slurry, such as to obtain a coverage of the slurry mixture corresponding to the desired coverage of the sponge part after crosslinking,

(g) Freeze-drying the resulting assembly obtained in (f) and crosslinking the freeze-dried product in a solution containing a crosslinking agent, washing with water and freeze-drying, and

(h) Optionally sterilizing the freeze-dried product obtained in (f) by ethylene oxide sterilization, $\gamma$ irradiation or X-ray irradiation.

14. A process according to claim 13, comprising:

- (a1) Preparing a slurry of denaturized collagen fibers derived from porcine peritoneum by submitting a porcine or bovine peritoneum or pericardium membrane to a basic treatment in a sodium hydroxide solution at a pH above 12, an acid treatment in a hydrochloric solution of pH of 1 to 3, a dehydrating treatment with a water soluble organic solvent and a degreasing treatment with an organic solvent, milling the dry membrane obtained with a cutting mill, sieving through a 0.5-2 mm sieve, acidifying the resulting collagen fibers in a slurry with repeated high pressure homogenization,
- Adding to the slurry mixture prepared in (f), 2 to 10 parts of the slurry prepared in (a1).

15. A process according to claim 13 or 14, wherein the crosslinking agent is selected from the group consisting of gluteraldehyde, EDC (1-ethyl-3-(3-dimethylaminopropyl)-carbodiimide) and a mixture of EDC and NHS (N-hydoxysuccinimide).

Fig. 1:

Chondro-Gide                    Alloderm                    Ossix Volumax

Fig. 2

A                    B                    C

FIG.3

35 mm

26 mm

10 mm

10 mm    10 mm

8 mm

10 mm

45 mm

22 mm

A          B    C          D

Fig. 4 :

Fig. 5 :

Europäisches
Patentamt

European
Patent Office

Office européen
des brevets

**EUROPEAN SEARCH REPORT**

Application Number

**EP 22 15 0470**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/082138 A2 (GEISTLICH PHARMA AG [CH]; GEISTLICH PETER [CH]; SCHLOESSER LOTHAR [DE]) 22 July 2010 (2010-07-22) * page 2, lines 25-28; claims 1,4,11; examples 1-4 * | 5-7 | INV. A61L27/24 A61L27/36 A61L27/56 |
| X | US 9 655 997 B2 (GEISTLICH PHARMA AG [CH]) 23 May 2017 (2017-05-23) * claim 1; examples 1-6 * | 1-4,8-12 | |
| Y | DAVID GIBEAULT J ET AL: "Use of cross-linked bovine pericardium as a disc replacement in the rabbit temporomandibular joint", JOURNAL OF ORAL AND MAXILLOFACIAL SURGERY, SAUNDERS, PHILADELPHIA, PA, US, vol. 47, no. 8, 1 August 1989 (1989-08-01), pages 828-833, XP027055060, ISSN: 0278-2391 [retrieved on 1989-08-01] * Introduction part; table 2 * | 5-7 | |

TECHNICAL FIELDS
SEARCHED (IPC)

**A61L**

The present search report has been drawn up for all claims

| Place of search | Date of completion of the search | Examiner |
|---|---|---|
| The Hague | 15 June 2022 | Siebum, Bastiaan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 0470

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2022

| Patent document cited in search report | | Publication date | Patent family member(s) | | Publication date |
|---|---|---|---|---|---|
| WO 2010082138 | A2 | 22-07-2010 | CA | 2749858 A1 | 22-07-2010 |
| | | | CA | 2749888 A1 | 22-07-2010 |
| | | | EP | 2387375 A1 | 23-11-2011 |
| | | | EP | 2387424 A2 | 23-11-2011 |
| | | | JP | 5698678 B2 | 08-04-2015 |
| | | | JP | 5698680 B2 | 08-04-2015 |
| | | | JP | 2012515035 A | 05-07-2012 |
| | | | JP | 2012515066 A | 05-07-2012 |
| | | | RU | 2011133948 A | 27-02-2013 |
| | | | RU | 2011133950 A | 27-02-2013 |
| | | | US | 2011270394 A1 | 03-11-2011 |
| | | | US | 2011274756 A1 | 10-11-2011 |
| | | | WO | 2010082138 A2 | 22-07-2010 |
| | | | WO | 2010083487 A1 | 22-07-2010 |
| US 9655997 | B2 | 23-05-2017 | AU | 2015367710 A1 | 29-06-2017 |
| | | | BR | 112017011140 A2 | 26-12-2017 |
| | | | CA | 2970849 A1 | 23-06-2016 |
| | | | CN | 106999632 A | 01-08-2017 |
| | | | DK | 3055000 T3 | 06-02-2017 |
| | | | EP | 3034103 A1 | 22-06-2016 |
| | | | EP | 3055000 A1 | 17-08-2016 |
| | | | ES | 2613672 T3 | 25-05-2017 |
| | | | HK | 1221668 A1 | 09-06-2017 |
| | | | HU | E031528 T2 | 28-07-2017 |
| | | | IL | 252909 A | 31-05-2018 |
| | | | JP | 6328861 B2 | 23-05-2018 |
| | | | JP | 2017537764 A | 21-12-2017 |
| | | | KR | 20170073709 A | 28-06-2017 |
| | | | MX | 368544 B | 07-10-2019 |
| | | | PL | 3055000 T3 | 31-05-2017 |
| | | | PT | 3055000 T | 07-02-2017 |
| | | | RU | 2672593 C1 | 16-11-2018 |
| | | | SG | 11201704864Y A | 28-07-2017 |
| | | | TW | 201627015 A | 01-08-2016 |
| | | | US | RE48536 E | 27-04-2021 |
| | | | US | 2016166737 A1 | 16-06-2016 |
| | | | WO | 2016096831 A1 | 23-06-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0324852 B1
- US 5007934 A
- US 6042610 A
- US 5837278 A
- US 5336616 A

### Non-patent literature cited in the description

- **STEINWACHS, M. R. et al.** Systematic Review and Meta-Analysis of the Clinical Evidence on the Use of Autologous Matrix-Induced Chondrogenesis in the Knee. *Cartilage,* 2019
- **K. et al.** Complex Meniscus Tears Treated with Collagen Matrix Wrapping and Bone Marrow Blood Injection: Clinical Effectiveness and Survivorship after a Minimum of 5 Years' Follow-Up. *Cartilage,* 2020
- **WANG, J. et al.** Characterization of regional meniscal cell and chondrocyte phenotypes and chondrogenic differentiation with histological analysis in osteoarthritic donor-matched tissues. *Sci Rep,* 2020, vol. 10 (1), 21658
- **VAN TIENEN, T. G. et al.** Tissue ingrowth and degradation of two biodegradable porous polymers with different porosities and pore sizes. *Biomaterials,* 2002, vol. 23 (8), 1731-1738
- **ZHANG, Z. Z. et al.** Role of scaffold mean pore size in meniscus regeneration. *Biomater,* 2016, vol. 43, 314-326
- **DELGADO, L. M. et al.** To cross-link or not to cross-link? Cross-linking associated foreign body response of collagen-based devices. *Tissue Eng Part B Rev,* 2015, vol. 21 (3), 298-313
- **BOEKEMA B.K.L.H. et al.** *Journal of Material Sciences: Materials in Medicine,* February 2014, vol. 25, 423-433
- **LEE, C. H. et al.** Protein-releasing polymeric scaffolds induce fibrochondrocytic differentiation of endogenous cells for knee meniscus regeneration in sheep. *Sci Transl Med,* 2014, vol. 6 (266), 266-171